# EUROPEAN PATENT APPLICATION

(11) **EP 1 528 611 A2**
(43) Date of publication of application: **04.05.2005**
(21) Application number: 04025420.3
(22) Date of filing: 26.10.2004
(51) Int. Cl.: H01L 51/30, C07D 271/06

(54) **White light-emitting compound, process for producing the same and luminescent element utilizing the same**

(30) Priority: 28.10.2003 JP 2003368155
(71) Applicant: HIROSE ENGINEERING CO., LTD., Tokyo 146-0092 (JP)
(72) Inventor: Nakaya, Tadao, Ohta-ku Tokyo 146-0092 (JP); Matsumoto, Ryoji, Ohta-ku Tokyo 146-0092 (JP)
(74) Representative: Klingseisen, Franz

(57) **Abstract**

The present invention provides a compound which is capable of emitting white light by itself and applicable, for example, to organic EL elements, a process for producing the compound, and a luminescent element utilizing the compound. The white light-emitting compound has the structure represented by formula (1):

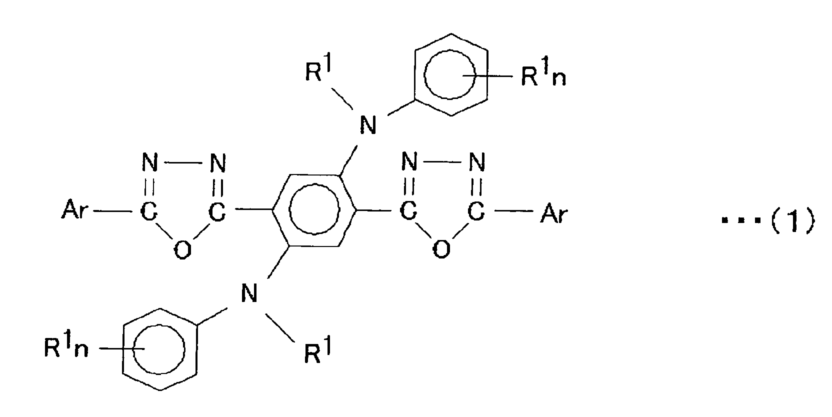

## Description

### Technical Field

The present invention relates to a white light-emitting compound, a process for producing the same, and a luminescent element utilizing the same. More particularly, this invention relates to a white light-emitting compound which is a novel compound capable of emitting white light by itself, a process for producing it, and a luminescent element utilizing it.

### Background Art

Developments of organic EL elements have been centered on those of elements respectively emitting lights, the colors of which are three primary colors, red (R) , green (G) and blue (B), and white light-emitting elements. The emission of white light was realized typically through mixing plural lights each having different colors. This technology is disclosed in JP63-19796, A.

However, few compounds that emit white light by themselves are known.

### Summary of the Invention

One objective of the present invention is to provide a compound which is capable of emitting white light by itself and applicable, for example, to organic EL elements, a process for producing the compound, and a luminescent element utilizing the compound. Another objective of the present invention is to provide an organic compound which is capable of emitting white light and applicable to various kinds of white light illuminators including organic EL elements. As a result of intensive studies to achieve the objectives, the inventors succeeded in synthesizing a single fluorescent compound capable of emitting white light at high purity, which led to the invention of a long-life EL element.

In order to achieve the objectives, the present invention provides a white light-emitting compound having the structure represented by formula (1). 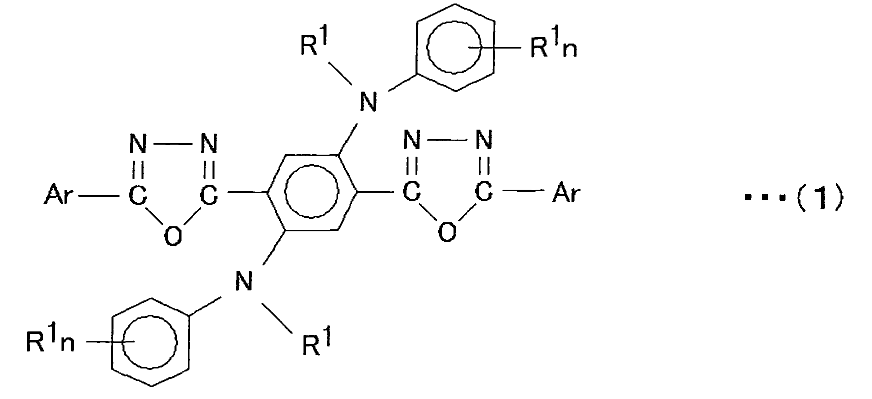 In formula (1), R¹ is hydrogen atom, an alkyl group having 1 to 10 carbon atoms, hydrogen atoms of which may be replaced with fluorine atoms, an alkoxyl group, or an aryl group; Ar denotes the substituent shown by one of formulas (1-1) to (1-6) below; and n is an integer from 1 to 5, wherein the number of R¹' s which one of the two phenyl groups in formula (1) has is the same as that of R¹' s which the other phenyl group has, and R¹ or R¹' s on one phenyl group are the same as R¹ or R¹' s on the other phenyl group; R¹ bonded with one of the two nitrogen atoms which are bonded with the respective phenyl groups mentioned above and the central benzene ring is the same as R¹ bonded with the other nitrogen atom; two Ar's are the same; and the R¹'s bonded with the nitrogen atoms may be different from those that the phenyl groups have.

Formula (1-1) is: 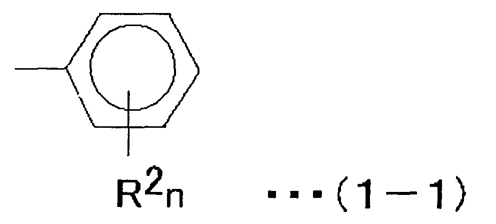 wherein R² means hydrogen atom, or an alkyl group having 1 to 10 carbon atoms, hydrogen atoms of which may be replaced with fluorine atoms; and n denotes the same as above.

Formula (1-2) is: 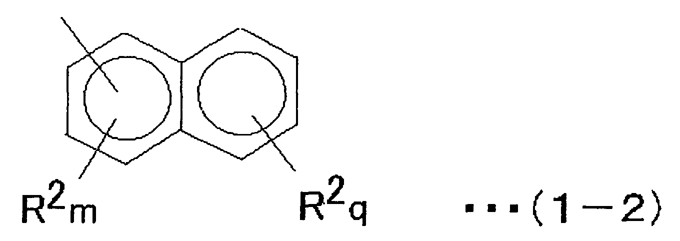 wherein R² means the same as above; m denotes an integer from 1 to 3; q denotes an integer from 1 to 4; and R²'s may be the same or different from each other.

Formula (1-3) is: 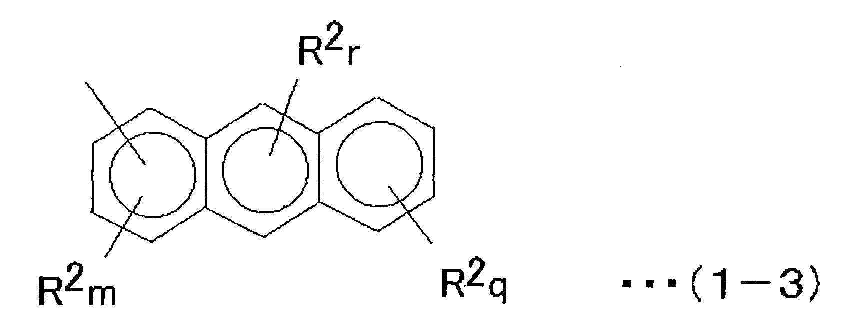 wherein R², m, and q mean the same as above; r denotes an integer of 1 or 2; and R²' s may be the same or different from each other.

Formula (1-4) is: 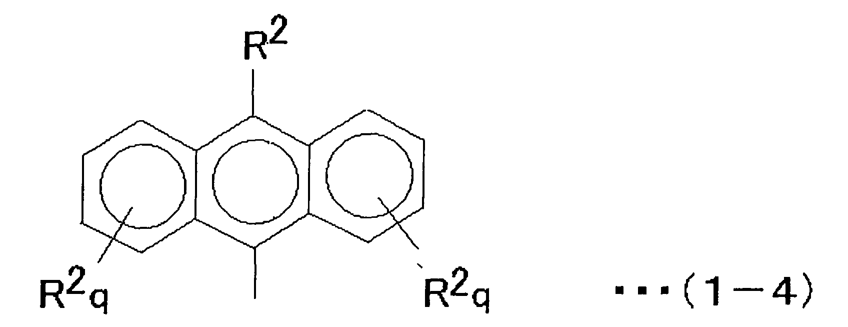 wherein R² and q mean the same as above; and R²' s may be the same or different from each other.

Formula (1-5) is: 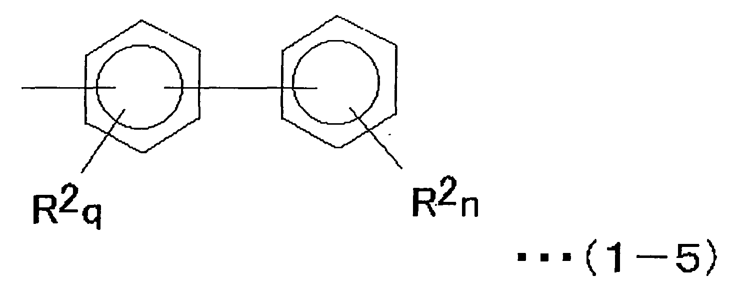 wherein R², n, and q mean the same as above; and R²'s may be the same or different from each other.

Formula (1-6) is: 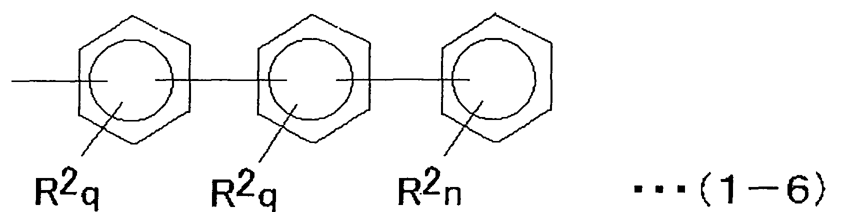 wherein R², n, and q mean the same as above; and R²'s may be the same or different from each other.

The second means provided by the present invention to achieve the objectives is a process for producing the white light-emitting compound which comprises:
preparing a first compound (c), in accordance with Reaction Formula 1, 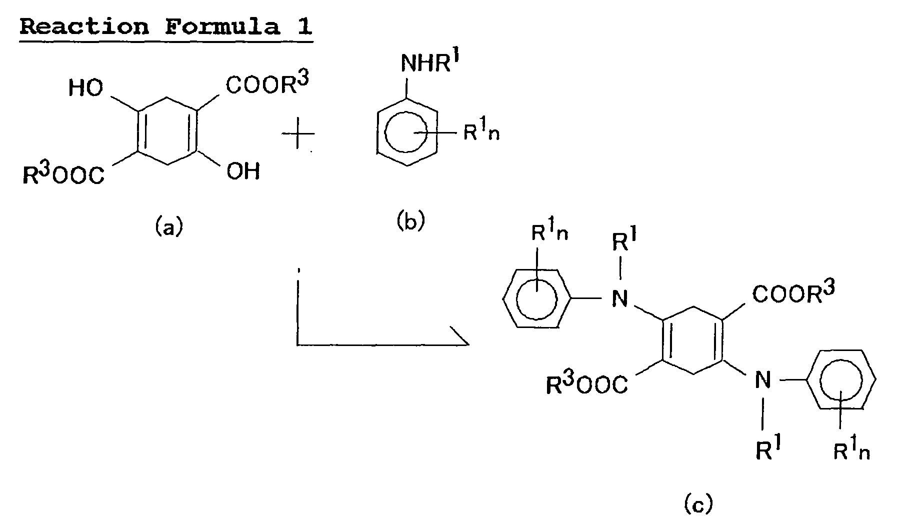 by heating 1,4-dihydroxy-2,5-dialkylcarboxy-2,4-cyclohexadiene (a) , wherein R³ of the compound (a) means an alkyl group, and an aniline derivative (b), wherein R¹ and n of the aniline derivative (b) are the same as those mentioned above, in a solvent, which results in the elimination of a water molecule made from the hydrogen atom bonded to the nitrogen atom of the aniline derivative and the hydroxyl group that 1,4-dihydroxy-2,5-dialkylcarboxy-2,4-cyclohexadiene (a) has;
preparing a second compound (d), in accordance with Reaction Formula 2, 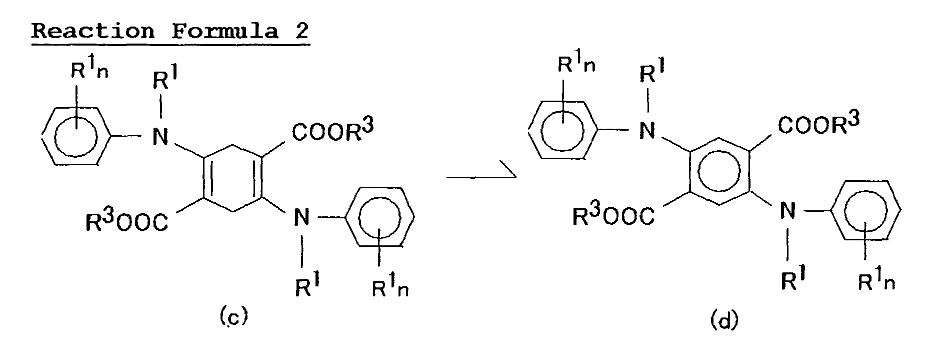 by heating the first compound (c) in a solvent in the presence of a dehydrogenating catalyst to subject the first compound (c) to the dehydrogenation reaction;
preparing a dicarboxylic acid compound (e) , in accordance with Reaction Formula 3, 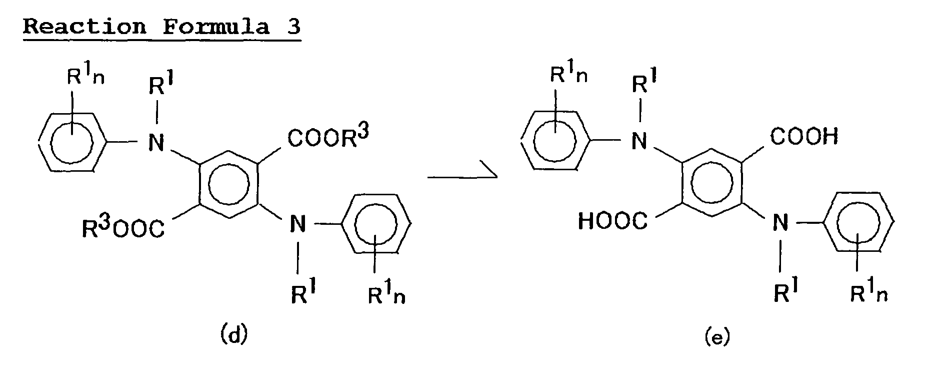 by heating the second compound (d) in a solvent, which replaces R³' s of compound (d) with hydrogen atoms;
preparing an acid halide (f), in accordance with Reaction Formula 4, 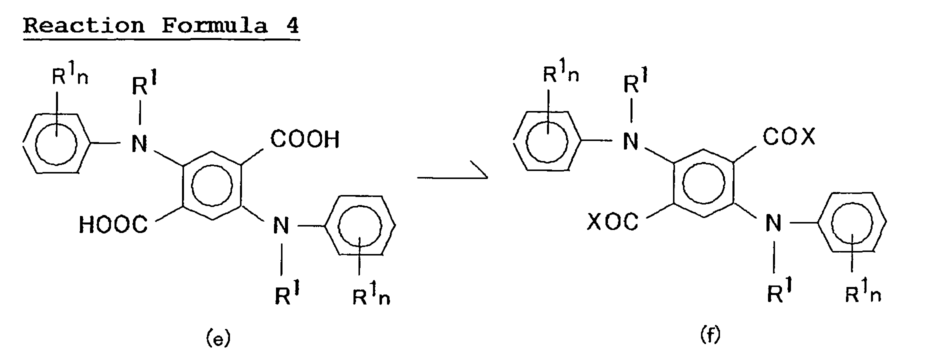 byheatingthedicarboxylicacidcompound (e) withahalogenating agent in a solvent, which replaces the hydroxyl groups of the carboxylic groups with halogen atoms (X);
preparing a third compound (g), in accordance with Reaction Formula 5, 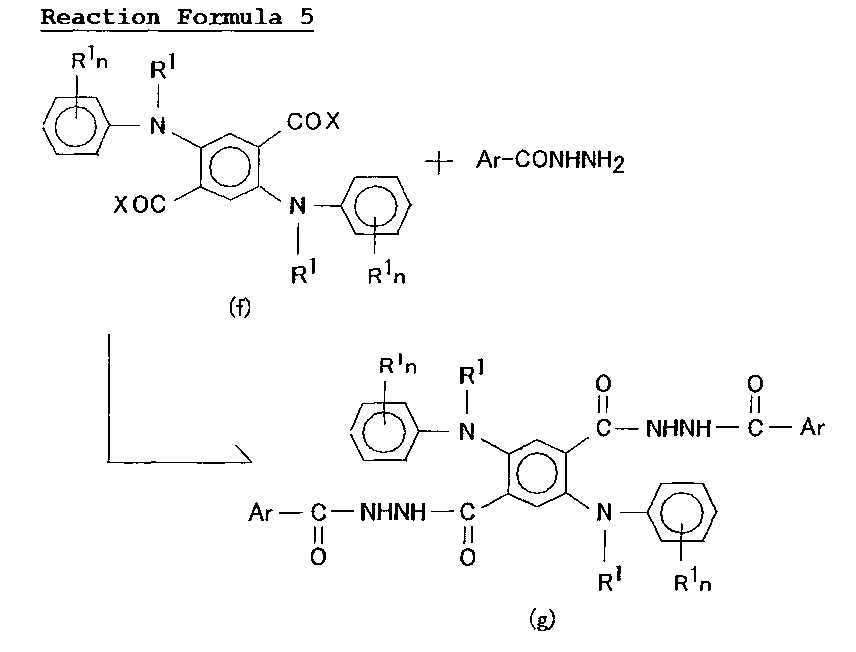 from the acid halide (f) and a hydrazide in which Ar means the same as that described in relation to formula (1); and
preparing a white light-emitting compound (h), in accordance with Reaction Formula 6, 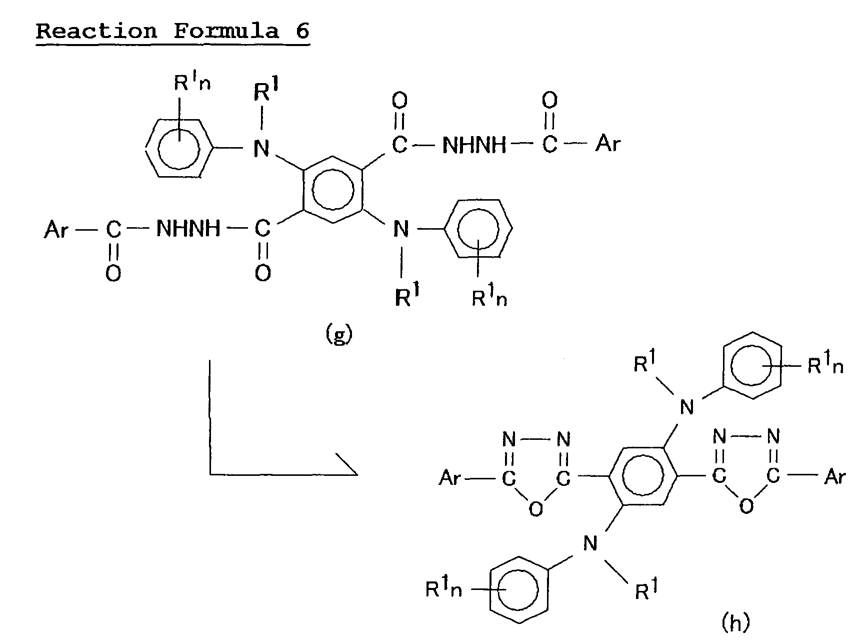 by heating the third compound (g) in a solvent, which makes the rings in the molecule.

The third means provided by the present invention is a luminescent element having a pair of electrodes and a light-emitting layer sandwiched between the electrodes, the light-emitting layer including the white light-emitting compound represented by formula (1).

The white light-emitting compound of the present invention has a centrosymmetrical structure, and has nitrilo groups and oxadiazole rings each having aryl groups. The oxadiazole rings having aryl groups are electron attractive, while the nitrilo groups are electron donative. Therefore, in the white light-emitting compound of the present invention, the π electron concentration of the oxadiazole rings is high, while that of the nitrilo groups is low. It is supposed that the parts, the π electron concentration of which is high, emit green light, that the parts, the π electron concentration of which is low, emit blue light, and that white light is made by the interference between the green light and the blue light. As a result of the interference, the human eye recognizes the color of the light emitted by the compound as white.

### Brief Description of the Drawings

Figure 1 is an illustration showing an example of the luminescent element in accordance with the present invention.
Figure 2 is an illustration showing another example of the luminescent element in accordance with the present invention.
Figure 3 is an illustration showing a still another example of the luminescent element in accordance with the present invention.
Figure 4 is an illustration showing a further example of the luminescent element in accordance with the present invention.
Figure 5 is an NMR spectrum chart of compound (4) having the structure represented by formula (4).
Figure 6 is an IR spectrum chart of compound (4) having the structure represented by formula (4).
Figure 7 is an NMR spectrum chart of compound (5) having the structure represented by formula (5).
Figure 8 is an IR spectrum chart of compound (5) having the structure represented by formula (5).
Figure 9 is an NMR spectrum chart of compound (9) having the structure represented by formula (9).
Figure 10 is an IR spectrum chart of compound (9) having the structure represented by formula (9).
Figure 11 is a fluorescence spectrum chart of compound (9) having the structure represented by formula (9).

### Detailed Description of the Preferred Embodiments

The white light-emitting compound according to the present invention is characterized by having the structure represented by formula (1).

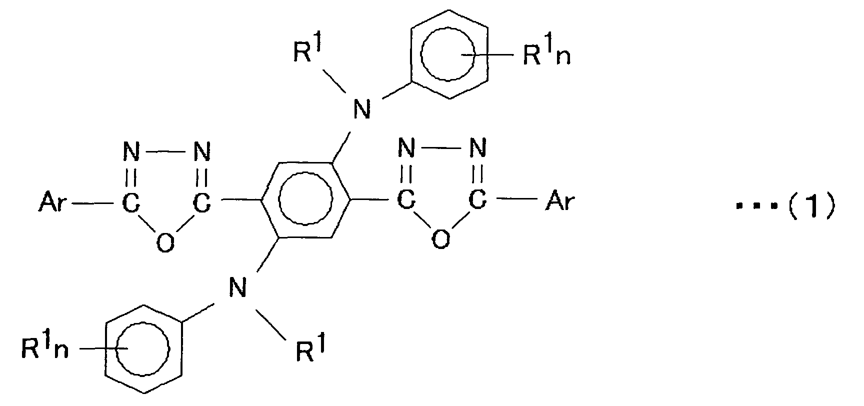

The compound represented by formula (1) has three benzene rings, two oxadiazole rings, two nitrogen atoms, two Ar's and several R¹' s.

The two oxadiazole rings are bonded with one of the three benzene rings so that the two oxadiazole rings are symmetrical around the benzene ring with which they are bonded. The two nitrogen atoms are bonded with the same benzene ring so that they are symmetrical around the benzene ring. The two oxadiazole rings each are also bonded with Ar's. One of the two nitrogen atoms is bonded with another one of the three benzene rings and a R¹, and the other one of the two nitrogen atoms is bonded with the other one of the three benzene rings and another R¹. The latter two benzenes respectively have R¹'s bonded.

"n" in formula (1) denotes the number of R¹' s replacing hydrogen atoms of each of the terminal benzene rings, or the phenyl groups. Specifically, "n" is an integer of 1 to 5. Therefore the compound represented by formula (1) has (2n + 2) R¹'s in total and the maximum number of R¹' s may be 12.

R¹ is hydrogen atom, an alkyl group having 1 to 10 carbon atoms, hydrogen atoms of which may be replaced with fluorine atoms, an alkoxyl group, or an aryl group.

Examples of the alkyl group having 1 to 10 carbon atoms, hydrogen atoms of which are not replaced with fluorine atoms, are methyl group, ethyl group, propyl group, isopropyl group, n-butyl group, isobutyl group, sec-butyl group, tert-butyl group, n-pentyl group, sec-pentyl group, tert-pentyl group, n-hexyl group, n-heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, and tert-pentyl group. Methyl group, ethyl group, and propyl group are especially preferable.

Examples of the alkyl group having 1 to 10 carbon atoms, hydrogen atoms of which are replaced with fluorine atoms, are fluoromethyl group, difluoromethyl group, trifluoromethyl group, fluoroethyl group, 1,1-difluoroethyl group, 1,2-difluoroethyl group, 1,1,1-trifluoroethyl group, 1,1,2-trifluoroethyl group, 1,2,2-trifluoroethyl group, 1,1,2,2-tetrafluoroethyl group, 1,1,2,2,2-pentafluoroethyl group, 1-fluoropropyl group, 2-fluoropropyl group, 1,1-difluoropropyl group, 1,2-difluoropropyl group, 1,3-difluoropropyl group, 2,2-difluoropropylgroup, 1,1,1-trifluoropropylgroup, 1,1,2-trifluoropropyl group, 1,2,3-trifluoropropyl group, 1,2,2-trifluoropropylgroup, and 1,3,3-trifluoropropyl group.

The alkoxyl groupmay include, for example, methoxyl group, ethoxyl group, propoxyl group, butoxyl group, isobutoxyl group, s-butoxyl group, and t-butoxyl group. Among them, methoxyl group, ethoxyl group, and propoxyl group are preferable.

Examples of the aryl group may be phenyl group, a tolyl group, a xylyl group, mesityl group, a cumenyl group, a biphenylyl group, a naphthyl group, an anthryl group, a phenanthryl group, etc.

R¹ or R¹' s on one of the two phenyl groups in formula (1) , or one terminal benzene ring, of the white light-emitting compound in accordance with the present invention are the same as R¹ or R¹' s on the other phenyl group, or the other terminal benzene ring. Also, the number of R¹'s which one phenyl group has is the same as that of R¹'s which the other phenyl group has.

In formula (1), Ar means the aryl group represented by one of formulas (1-1) to (1-6) below.

Formula (1-1) is:

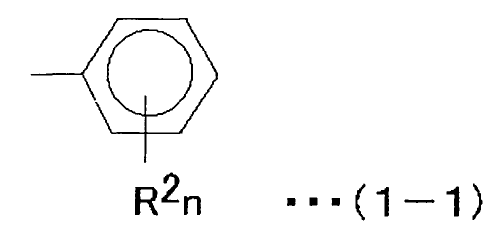

The aryl group shown by formula (1-1) has a phenyl skeleton, which has at least one R².

R² means hydrogen atom, or an alkyl group having 1 to 10 carbon atoms, hydrogen atoms of which may be replaced with fluorine atoms. Examples of the alkyl group have been listed above. Also, n denotes the same as above. When n is one of 2 to 5, R²'s may be the same or different from each other.

Formula (1-2) is:

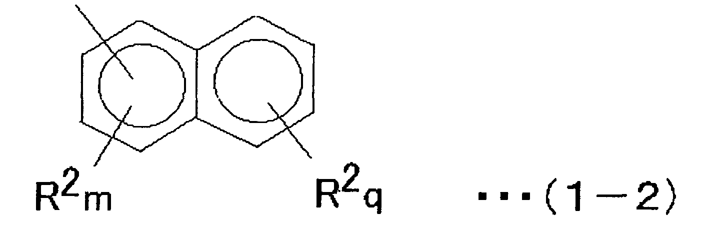

The aryl group represented by formula (1-2) has a 1-naphthyl or 2-naphthyl skeleton, and has substituents R². R² means the same as above, m denotes an integer from 1 to 3, and q denotes an integer from 1 to 4. R²' s may be the same or different from each other.

Formula (1-3) is:

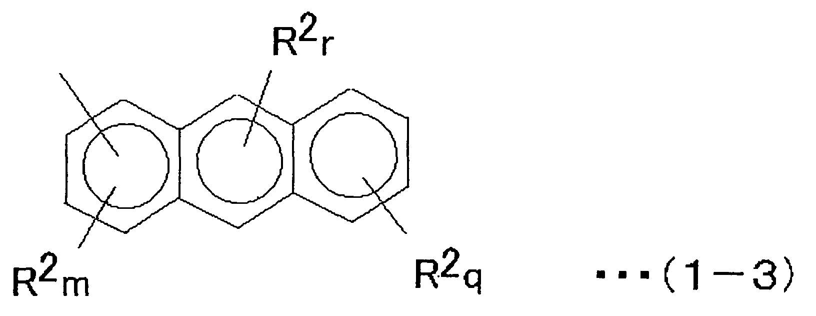

The aryl group represented by formula (1-3) has a 1-anthryl or 2-anthryl skeleton, and has substituents R². R², m, and q in formula (1-3) mean the same as above, and r denotes an integer of 1 or 2. R²'s may be the same or different from each other.

Formula (1-4) is:

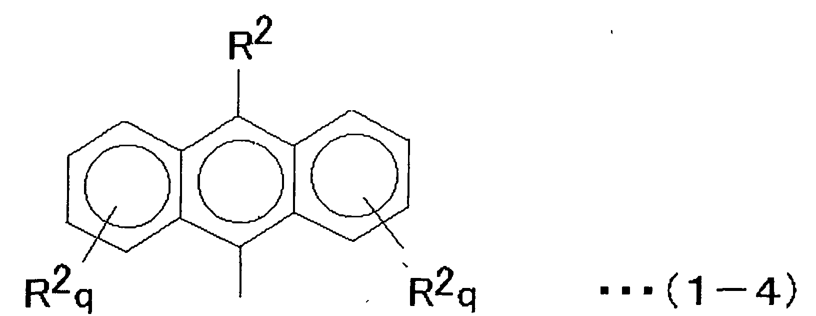

Thearyl group represented by formula (1-4) has a 9-anthryl skeleton, and has substituents R². R² and q mean the same as above, and R²' s may be the same or different from each other.

Formula (1-5) is:

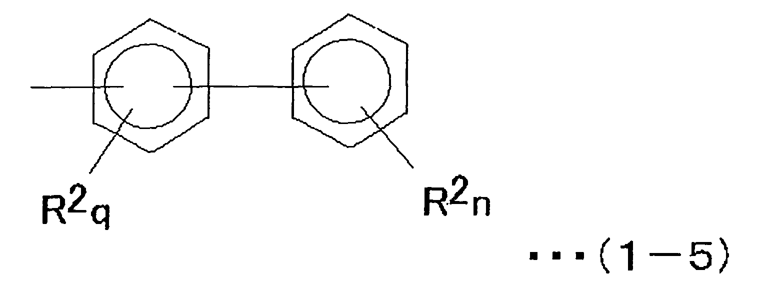

The aryl group represented by formula (1-5) has a 2-biphenylel, 3-biphenylyl, or 4-biphenylyl skeleton, and has substituents R². R², n, and q mean the same as above. R²' s may be the same or different from each other.

Formula (1-6) is:

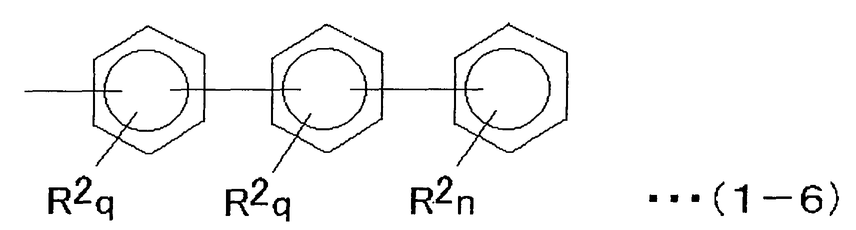

The aryl group represented by formula (1-6) has a 2-triphenylyl, 3-triphenylyl, or 4-triphenylyl skeleton, and has substituents R². R², n, and q mean the same as above. R²' s may be the same or different from each other.

The two Ar' s in formula (1) are the same aryl group. Note that the white light-emitting compound of the present invention is characterized by its cetrosymmetrical structure. Therefore, as long as this structural characteristic is not lost, the groups R¹'s and Ar's in formula (1) may be the same or different from each other.

The white light-emitting compound in accordance with the present invention has two electron donative macrogroups, each of which consists of the nitrogen atom, the terminal benzene ring, and the substituent R¹, and two electron attractive macrogroups, each of which consists of the oxadiazole ring and the substituent Ar. Also, the compound has another benzene ring at the center thereof. The two electron donative macrogroups are bonded with the central benzene ring so that they are centrosymmetrical around the benzene ring, and the two electron attractive macrogroups are bonded with the central benzene ring in the same way. Therefore, the white light-emitting compound in accordance with the present invention has a centrosymmetrical structure. Since the white light-emitting compound of the present invention has the electron donative macrogroups and the electron attractive macrogroups as mentioned above, the compound has the parts where the π electron concentration is high and those where the concentration is low. It is supposed that the parts, the π electron concentration of which is high, emit green light, that the parts, the π electron concentration of which is low, emit blue light, and that white light is made by the interference between the green light and the blue light. Therefore, the white light-emitting compound in accordance with the invention *per se,* which is a single compound, can emit white light.

The white light-emitting compound represented by formula (1) is prepared by the steps shown in Reaction Formulas 1 to 6 below.

First, a first compound (c) may be prepared, in accordance with Reaction Formula 1, 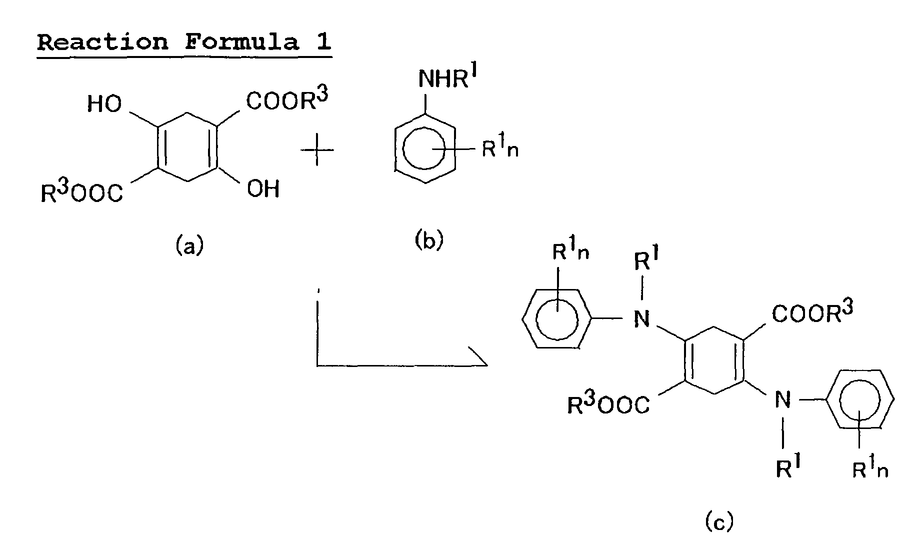 by heating 1,4-dihydroxy-2,5-dialkylcarboxy-2,4-cyclohexadiene (a) and an aniline derivative (b) in a solvent, which results in the elimination of a water molecule made from the hydrogen atom bonded to the nitrogen atom of the aniline derivative and the hydroxyl group that 1,4-dihydroxy-2,5-dialkylcarboxy-2,4-cyclohexadiene (a) has.

In Reaction Formula 1, R³ of 1,4-dihydroxy-2,5-dial kylcarboxy-2, 4-cyclohexa-diene (a) denotes an alkyl group, and R¹ of the aniline derivative (b) denotes the same group as that explained in relation to formula (1).

The solvent may be a non-polar solvent such as benzene, or a polar solvent such as methanol, ethanol, acetic acid, phthalic acid, phthalic anhydride, glacial acetic acid, ortho-dichlorobenzene, meta-dichlorobenzene, dioxane, _pyridine, N,N-dimethylformamide, and tetrahydrofuran (THF). These solvents may be used alone or in combination.

The reaction temperature should be 80 to 130°C, particularly 90 to 110°C.

The heating time should be 5 to 7 hours, particularly 5 to 6 hours.

Then, a second compound (d) may be prepared, in accordance with Reaction Formula 2, 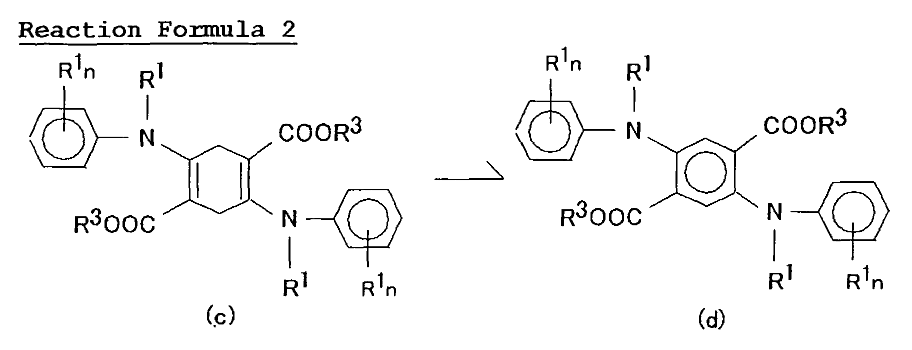 by heating the first compound (c) in a solvent in the presence of a dehydrogenating catalyst to subject the first compound (c) to the dehydrogenation reaction.

The dehydrogenating catalyst may be an acid catalyst, such as sulfuric acid, nitric acid, or hydrochloric acid; a metal catalyst, such as chromium, iron, palladium, silver, or copper; or an acidic oxide, such as aluminum oxide or magnesium oxide.

The solvent may be a non-polar solvent such as benzene, or a polar solvent such as methanol, ethanol, acetic acid, phthalic acid, phthalic anhydride, glacial acetic acid, ortho-dichlorobenzene, meta-dichlorobenzene, dioxane, pyridine, N,N-dimethylformamide, and tetrahydrofuran (THF). These solvents may be used alone or in combination.

The reaction temperature should be 130 to 170°C, particularly 140 to 160°C.

The heating time should be 3 to 5 hours, particularly 3 to 4 hours.

Next, a dicarboxylic acid compound (e) may be prepared, in accordance with Reaction Formula 3, 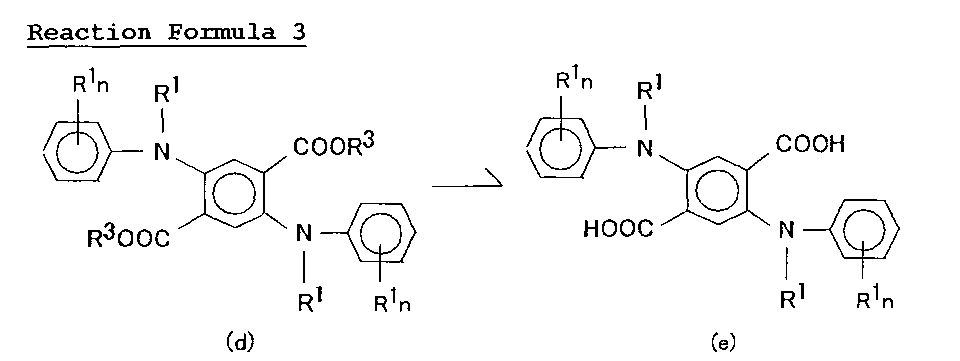 by heating the obtained compound (d) in a solvent, which replaces R³'s of compound (d) with hydrogen atoms.

The solvent may be a non-polar solvent such as benzene, or a polar solvent such as methanol, ethanol, acetic acid, phthalic acid, phthalic anhydride, glacial acetic acid, ortho-dichlorobenzene, meta-dichlorobenzene, dioxane, pyridine, N,N-dimethylformamide, and tetrahydrofuran (THF). These solvents may be used alone or in combination.

The reaction temperature should be 80 to 140°C, particularly 100 to 120°C.

The heating time should be 0.5 to 4 hours, particularly 1.5 to 2.5 hours.

This reaction can proceed only if the second compound (d) is heated in the solvent. However, in order to expedite the reaction, an acid such as hydrochloric acid or sulfuric acid, or an alkali such as sodium hydroxide or potassium hydroxide, as a catalyst, should be employed.

Furthermore, anacidhalide (f) isprepared, inaccordance with Reaction Formula 4, 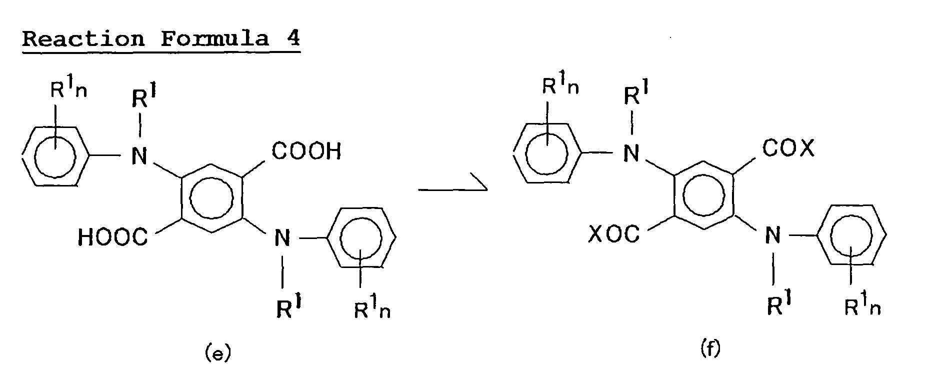 by heating the dicarboxylic acid compound (e) with a halogenating agent in a solvent, which replaces the hydroxyl groups of the carboxylic groups with halogen atoms (X).

The halogenating agent includes a chlorinating agent such as thionyl chloride or phosphoryl chloride, a brominating agent such as thionyl bromide, etc.

The solvent may be a non-polar solvent such as benzene, or a polar solvent such as methanol, ethanol, acetic acid, phthalic acid, phthalic anhydride, glacial acetic acid, ortho-dichlorobenzene, meta-dichlorobenzene, dioxane, pyridine, N,N-dimethylformamide, and tetrahydrofuran (THF). These solvents may be used alone or in combination.

The reaction temperature should be 80 to 140°C, preferably 90 to 120°C, particularly 100 to 110°C.

The heating time should be 1 to 4 hours, particularly 1 to 3.5 hours.

The halogen atoms of the acid halide (f), shown by Xs in Reaction Formula 5, each may be chlorine atoms, fluorine atoms, or bromine atoms.

Then, a third compound (g) may be prepared, in accordance with Reaction Formula 5, 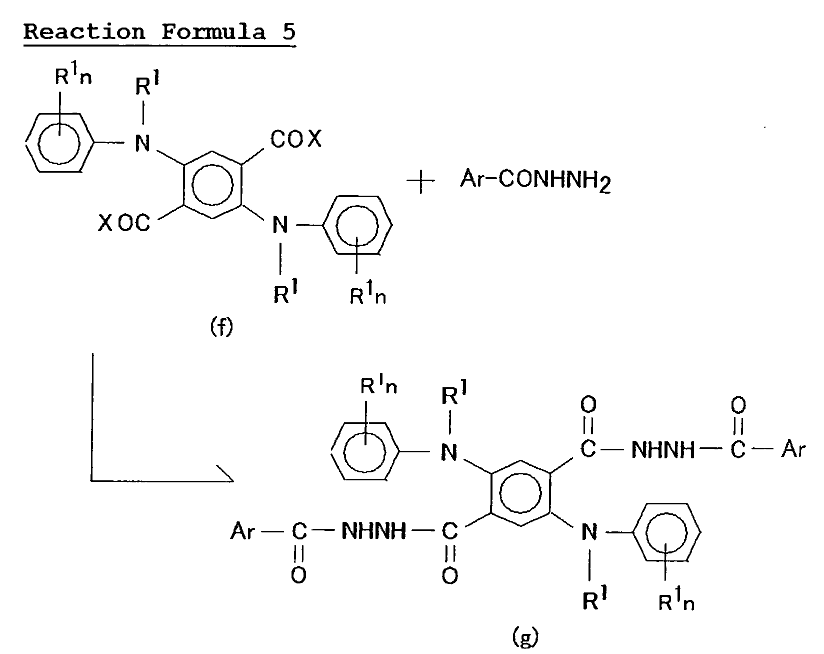 from the acid halide (f) and a hydrazide.

The solvent may be a non-polar solvent such as benzene, or a polar solvent such as methanol, ethanol, acetic acid, phthalic acid, phthalic anhydride, glacial acetic acid, ortho-dichlorobenzene, meta-dichlorobenzene, dioxane, pyridine, N,N-dimethylformamide, and tetrahydrofuran (THF). These solvents may be used alone or in combination.

The reaction temperature should be 40 to 80°C, particularly 50 to 60°C.

The heating time should be 1 to 4 hours, particularly 1 to 3 hours.

Finally, a white light-emitting compound (h) may be prepared, in accordance with Reaction Formula 6, 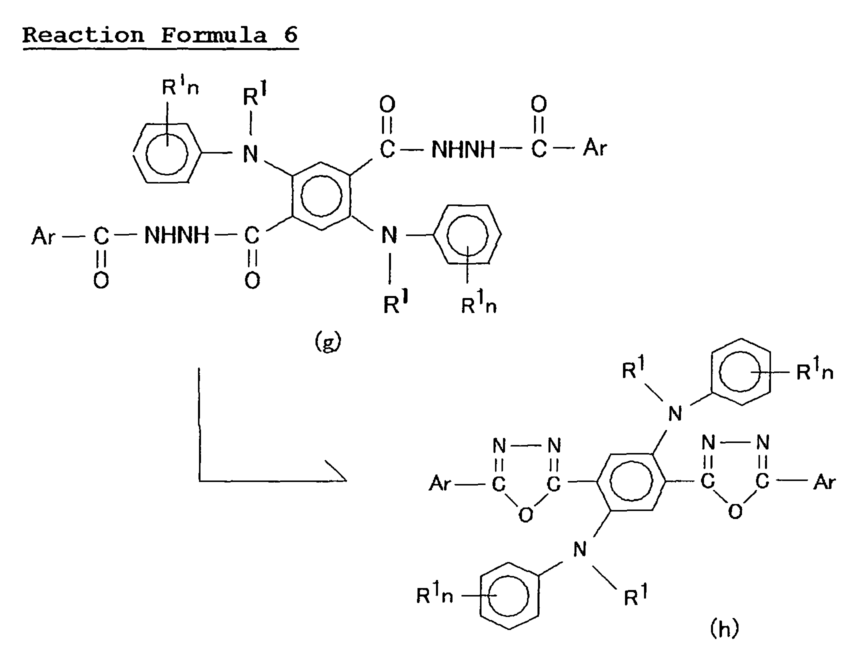 by heating the third compound (g) in a solvent, which makes the rings in the molecule.

The solvent may be a polar solvent such as phosphoryl chloride, phosphoryl bromide, ortho-dichlorobenzene, meta-dichlorobenzene, dioxane, pyridine, N,N-dimethylformamide, tetrahydrofuran (THF), etc. These solvents may be used alone or in combination.

The reaction temperature should be 100 to 140°C, particularly 90 to 120°C.

The heating time should be 6 to 24 hours, particularly 10 to 15 hours.

The white light-emitting compound-(h) can be obtained through purification and separation by known methods after the completion of the series of reactions.

The white light-emitting compound according to the present invention can easily be produced simply by heating. Therefore it can be said that this simple process of producing the white light-emitting compound is an industrial method.

The luminescent element of the present invention will be explained in the followings.

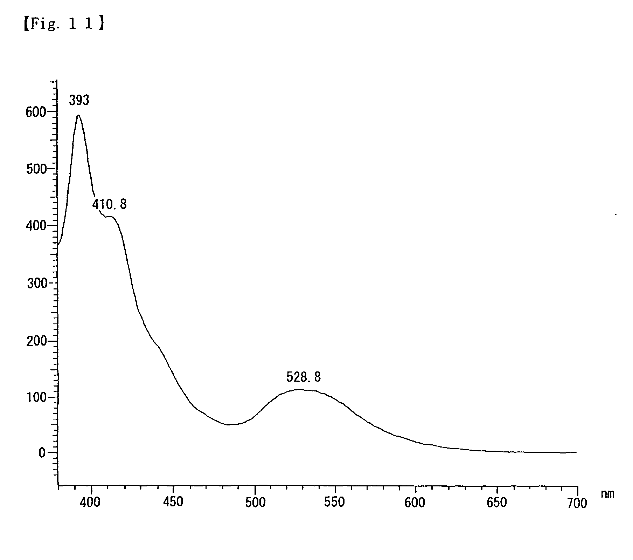

Upon the application of electromagnetic wave energy, it is observed that the white light-emitting compound of the present invention emits a visible light of which wavelength ranges 300 nm and 600 nm. They have a fluorescence spectrum, for example as shown in Figure 11, and may be utilized for an organic electroluminescent element.

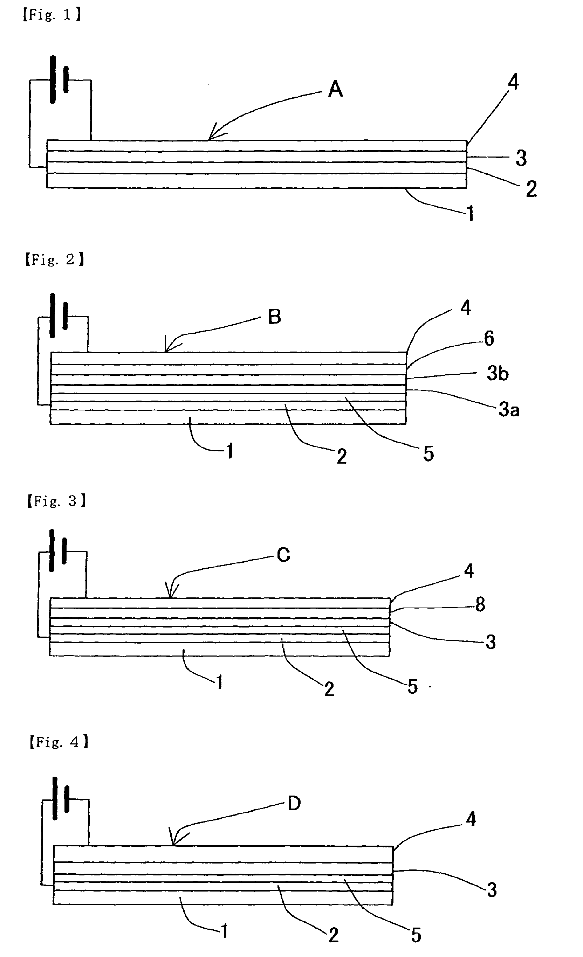

Figure 1 is a schematic illustration that shows the sectional structure of a luminescent element emitting white light, which is a one-layer type organic EL element. As shown in this figure, a luminescent element A is prepared by layering a light-emitting layer 3 and an electrode layer 4 in this order on a substrate 1 with which a transparent electrode 2 has been provided. The light-emitting layer 3 includes the white light-emitting compound.

When electric current is applied to the white light-emitting luminescent element A shown in Figure 1 at the transparent electrode 2 and the electrode layer 4, the element emits white light. Upon the application of an electric field between the transparent electrode 2 and the electrode layer 4, electrons are injected from the electrode layer 4 and positive holes from the transparent electrode 2. In the light-emitting layer 3, the electrons are recombined with positive holes, which causes the energy level to return to the valence band from the conduction band. This transition of the energy level is accompanied by emission of the energy differential as light.

In addition, because the luminescent element A includes a white light-emitting compound having the special structure in the light-emitting layer, it has a long life. Therefore, light sources employing the luminescent element Awill naturally have a long life.

The luminescent element Amay also be shaped into a tubular light emitter comprising a tubularly shaped substrate 1, a transparent electrode 2 placed on the inside surface of the substrate 1, a light emitting layer 3 and an electrode layer 4 placed on the transparent electrode 2 in this order.

For the substrate 1 may be used any known substrate, as long as the transparent electrode 2 can be formed on the surface of the substrate. Examples of the substrate 1 are a glass substrate, a plastic sheet, a ceramic substrate, and a metal substrate of which surface is insulated, for example, through the formation of an insulating layer thereon. When the substrate 1 is opaque, the luminescent element will be utilized as a single-sided illuminator which emits light only from the side on which the light-emitting layer is placed. When the substrate 1 is transparent, the element can be used as a double-faced illuminator which emits light from the both sides of the substrate 1.

For the transparent electrode 2, various materials may be employed, as long as their work functions are large, they are transparent, and they can function as a cathode and inject holes to the light-emitting layer 3 when voltage is applied thereto. Specifically, the transparent electrode 2 may be made of a transparent inorganic conductive material such as ITO, In₂O₃, SnO₂, ZnO, or CdO, or an electrically conductive high polymer such as polyaniline.

The transparent electrode 2 may be formed on the substrate 1 by chemical vapor phase deposition, spray pyrolysis, high-vacuum metal deposition, electron beam deposition, sputtering, ion beam sputtering, ion plating, ion-assisted deposition, and other methods.

When the substrate is made of an opaque material, the electrode formed on the substrate need not be transparent.

The light-emitting layer 3 is a layer that includes the white light-emitting compound represented by formula (1) in accordance with the present invention. The light-emitting layer 3 may be a high polymer film where the white light-emitting compound is dispersed in a high polymer. The layer may also be a deposited film prepared by depositing the white light-emitting compound on the transparent electrode 2.

Examples of the high polymer for the high polymer film are a polyvinyl carbazole, a poly (3-alkylthiophen) , a polyimide including an arylamide, a polyfluorene, a polyphenylene vinylene, a poly-α-methylstyrene, a copolymer of vinylcarbazole and α-methylstyrene. Among them, a polyvinyl carbazole is preferable.

The amount of the light-emitting compound included in the high polymer film is, typically 0.01-2 weight%, preferably 0.05-0.5 weight%.

The thickness of the high polymer film ranges, typically between 30 nm and 500 nm, preferably between 100 nm and 300 nm. When the thickness is too small, the amount of the emitted light may be insufficient. On the other hand, when the thickness is too large, the voltage required to drive the illuminator or element may be too high, which is not desirable. Besides, the large thickness may reduce the flexibility of the film necessary to shape a planar, tubular, curved, or ring article.

The high polymer filmmay be formed through the application of a solution of the high polymer and the light-emitting compound of the invention dissolved in a suitable solvent. The application method is one selected from a spin cast method, a coating method, a dipping method, etc.

When the light-emitting layer 3 is a deposited film, the thickness of the film is generally 0.1-100 nm, although a preferable thickness is different depending on the structure of layers and other factors. When the thickness is too large or too small, it might cause the same problems as described above.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode 4 may easily be formed on the surface of light-emitting layer 3, which, in turn, has been formed on substrate 1, by the technique of metal deposition.

When either of the deposition or the application is employed, a buffer layer should be inserted between each electrode and the light-emitting layer.

Materials for the buffer layer are, for example, an alkaline metal compound such as lithium fluoride, an alkaline earth metal compound such as magnesium fluoride, an oxide such as an aluminum oxide, and 4, 4 ' -biscarbazole biphenyl (Cz-TPD) . Also, materials for forming the buffer layer between the cathode made of a material such as ITO and the organic layer are, for example, m-MTDATA (4,4',4"-tris(3-methylphenyl-phenylamino) triphenylamine), phthalocyanine, polyaniline, and polythiophene derivatives, and inorganic oxides such as molybdenum oxide, ruthenium oxide, vanadium oxide and lithium fluoride. When the materials are suitably selected, these buffer layers can lower the driving voltage of the organic EL element, which is the luminescent element emitting white light, improve the quantum efficiency of luminescence, and achieve an increase in the luminance of the emitted light.

The second example of the luminescent element emitting white light in accordance with this invention is shown in Figure 2. This figure is an illustration showing the sectional layer structure of an example of the luminescent element, which is a multi-layer organic EL element.

As shown in Figure 2, the luminescent element B comprises a substrate 1, and a transparent electrode 2, a hole-transporting layer 5, light-emitting sublayers 3a and 3b, an electron-transporting layer 6, and an electrode layer 4, the layers being laid on the substrate 1 one by one in this order.

The substrate 1, the transparent electrode 2 and the electrode layer 4 are the same as those explained for the luminescent element A in Figure 1.

The light-emitting layer of the luminescent element B comprises light-emitting sublayers 3a and 3b. The light-emit ting sublayer 3a is a film including the white light-emitting compound of this invention. The light-emitting sublayer 3b is a DPVBi layer, which functions as a host material.

Examples of the hole-transporting substance included in the hole-transporting layer 5 are a triphenylamine compound such as N, N'-diphenyl-N, N'-di (m-tolyl)-benzidine (TPD) and α -NPD, a hydrazon compound, a stilbene compound, a heterocyclic compound, a π electron star burst positive hole transporting substance, etc.

Examples of the electron-transporting substance included in the electron-transporting layer 6 are an oxadiazole derivative such as 2-(4-tert-butylphenyl)-5-(4-biphenylyl)-1,3,4-oxadiazole and 2,5-bis(1-naphthyl)-1,3,4-oxadiazole (BND), and 2,5-bis(5'-tert-butyl-2'-benzoxazolyl) thiophene. Also, a metal complex material such as quinolinol aluminum complex (Alq3), benzoquinolinol beryllium complex (Bebq2) may be used suitably.

The electron-transporting layer 6 of the luminescent element B shown in Figure 2 includes Alq3 as electron-transporting substance.

The thickness of each layer is the same as that in a known multi-layer organic EL element.

The luminescent element B in Figure 2 functions and emits light in the same ways as the luminescent element A in Figure 1. Therefore, the luminescent element B has the same uses as the luminescent element A.

The third example of the luminescent element of this invention is shown in Figure 3. This figure is an illustration showing the sectional layer structure of an example of the luminescent element emitting white light, which is a multi-layer organic EL element.

The luminescent element emitting white light C shown in Figure 3 comprises a substrate 1, and a transparent electrode 2, a hole-transporting layer 5, a light-emitting layer 3, an electron-transporting layer 8, and an electrode layer 4, wherein the transparent electrode and the layers are laid on substrate 1 one by one in this order.

The luminescent element C functions in the same way as the luminescent element B.

Another example of the luminescent element of this invention is shown in Figure 4. The luminescent element emitting white light D comprises a substrate 1, and a transparent electrode 2, a hole-transporting layer 5, a light-emitting layer 3, and an electrode layer 4 wherein the transparent electrode and the layers are laid on the substrate 1 one by one in this order.

An example of the luminescent elements, other than those shown in Figures 1-4, is a two-layer low molecular weight organic luminescent element having a hole-transporting layer that includes a hole-transporting substance and an electron-transporting luminescent layer that includes the white light-emitting compound of the invention laid on the hole-transporting layer, these layers being sandwiched between a cathode, which is the transparent electrode formed on the substrate, and an anode, which is the electrode layer. A specific example of this embodiment is a two-layer pigment-injected luminescent element comprising a hole-transporting layer and a light-emitting layer that includes a host pigment and the white light-emitting compound of this invention as a guest pigment, wherein the light-emitting layer is laid on the hole-transporting layer and these layers are sandwiched between the cathode and the anode. Another example is a two-layer organic luminescent element comprising a hole-transporting layer that includes a hole-transporting substance and an electron-transporting luminescent layer that is prepared through a co-deposition of the white light-emitting compound of the invention and an electron-transporting substance, the latter layer being laid on the former, and these two layers being sandwiched between the cathode and the anode. A specific example of the second embodiment is a two-layer pigment-injected luminescent element comprising a hole-transporting layer and an electron-transporting luminescent layer that includes a host pigment and the white light-emitting compound of this invention as a guest pigment, wherein the luminescent layer is laid on the hole-transporting layer and these layers are sandwiched between the cathode and the anode. A further example is a three-layer organic luminescent element comprising a hole-transporting layer, a luminescent layer including the white light-emitting compound of this invention that is laid on the hole-transporting layer, and an electron-transporting layer that is laid on the luminescent layer, these layers being sandwiched between the cathode and the anode.

The electron-transporting luminescent layer, typically, comprises 50-80 weight% of polyvinylcarbazole, which is abbreviated to PVK, 5-40 weight% of an electrode-transporting luminescent agent, and 0.01-20 weight% of the white light-emitting compound of this invention. The electron-transporting luminescent layer of this composition is capable of emitting white light at a high luminance.

Also, it is preferable, if the light-emitting layer includes, as a sensitizing agent, rubrene, especially both of rubrene and Alq3.

An illuminator utilizing the white light-emitting compound of the present invention may generally be used as an organic EL element which is driven, generally, by direct current, and also by pulses and alternating current.

### Examples

### (Example 1)

In a 2000 ml three-necked flask were placed 50 g of 4-heptylaniline, 27.1 g of 1, 4 -dihydroxy-2, 5-dimethylcaroboxy-2,4-cyclohexadiene, 500 ml of ethanol, and 500 ml of acetic acid. The flask containing the mixture was placed in a silicone oil bath and the mixture was heated to 110°C. The reaction was allowed to continue for 20 hours. After the termination of the reaction, the solution in the flask was cooled naturally. The solution was filtered. The filtrate obtained by the filtration was washed with methanol and then dried up. 49 g of a red solid matter was obtained.

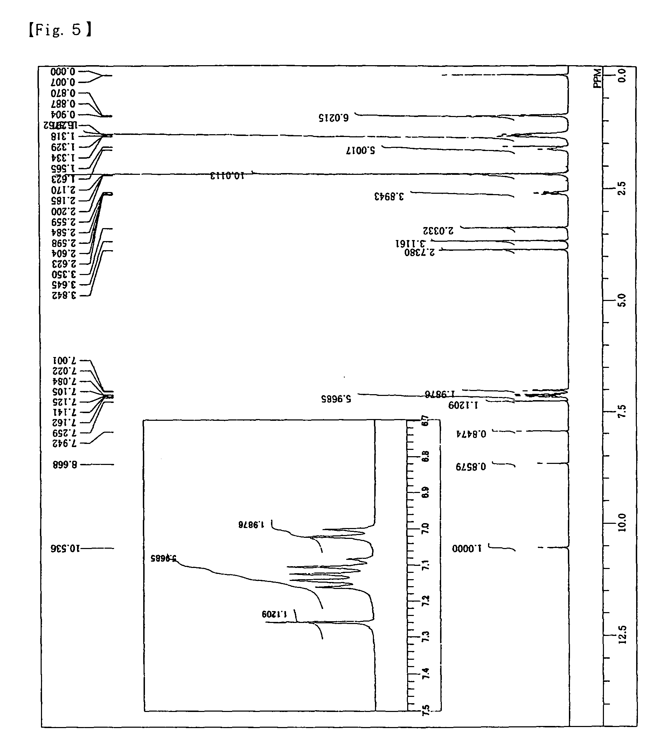

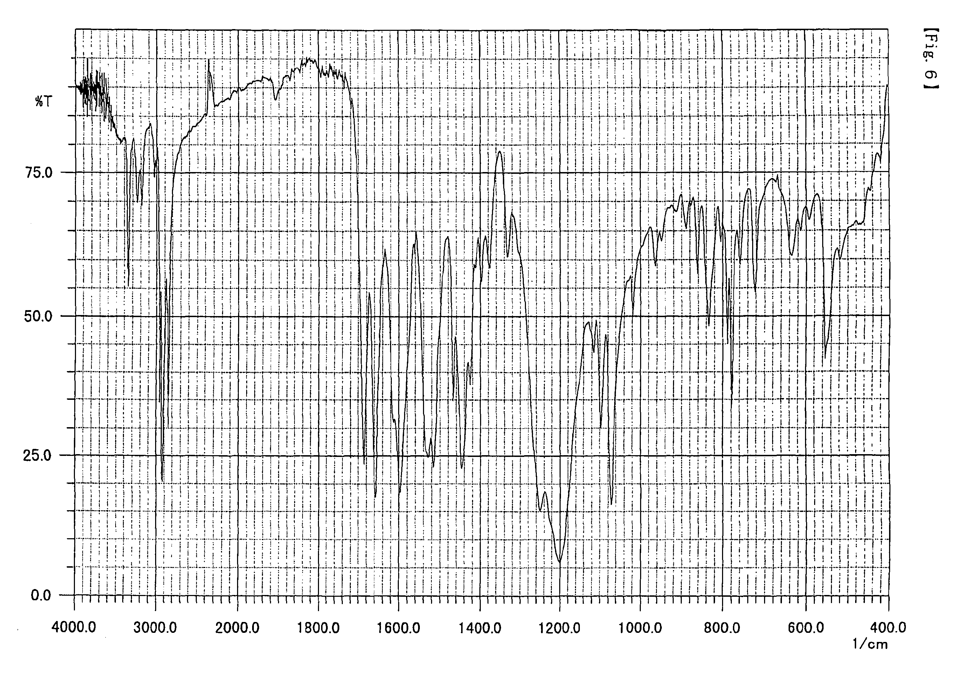

An NMR spectrum chart and an IR spectrum chart of the solid matter are respectively shown in Figure 5 and Figure 6. This solid matter was identified as a compound having the structure represented by formula (4) below.

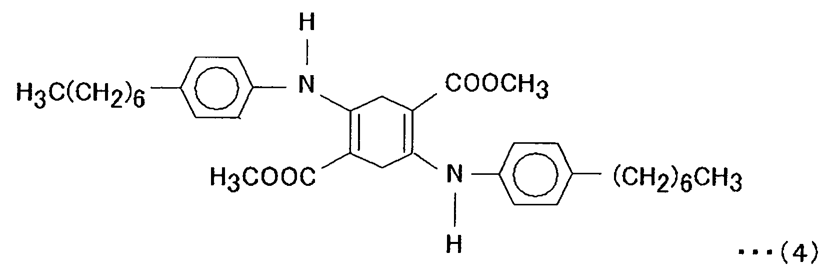

Next, in a 1000 ml three-necked flask, 25 g of the compound shown by formula (4), 600 ml of ortho-dichlorobenzene, and a proper amount of sulfuric acid were placed. The flask containing the mixture was placed in a silicone oil bath and the mixture was heated to 160°C. The reaction was allowed to continue for 1 hour. After the termination of the reaction, the resultant solution was cooled naturally. After the cooling, the solvent was distilled away. The remaining liquid was washed with methanol and dried up. 10.6 g of a solid matter was obtained.

In a 1000 ml three-necked flask, 10.6 g of the obtained solid matter, 600 ml of ortho-dichlorobenzene, and a proper amount of sulfuric acid were placed. The flask containing the mixture was placed in a silicone oil bath and the mixture was heated to 160°C. The reaction was allowed to continue for 1 hour. After the termination of the reaction, the resultant solution was cooled naturally. After the cooling, the solvent was distilled away. The remaining liquid was washed with methanol and dried up. 8.4 g of a solid matter was obtained.

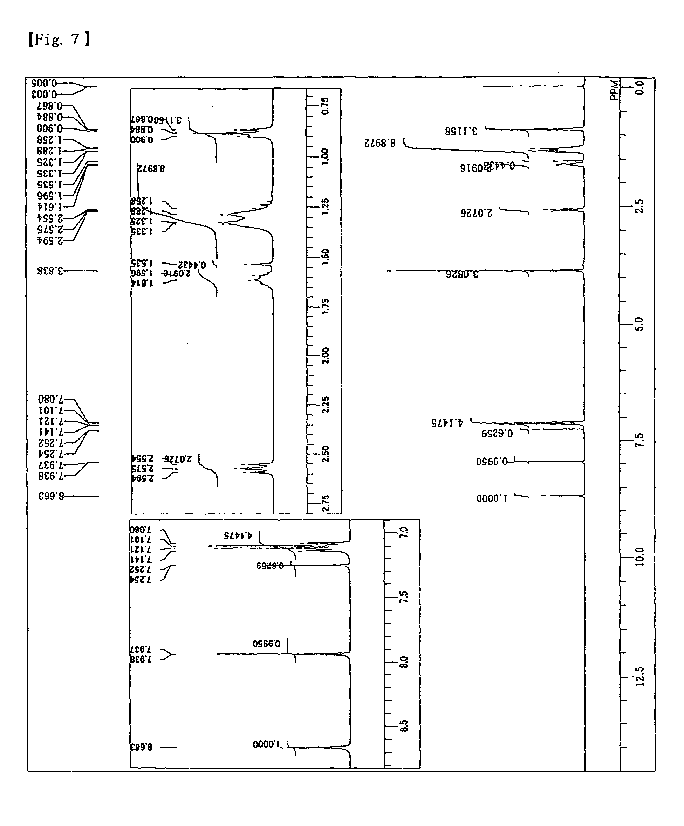

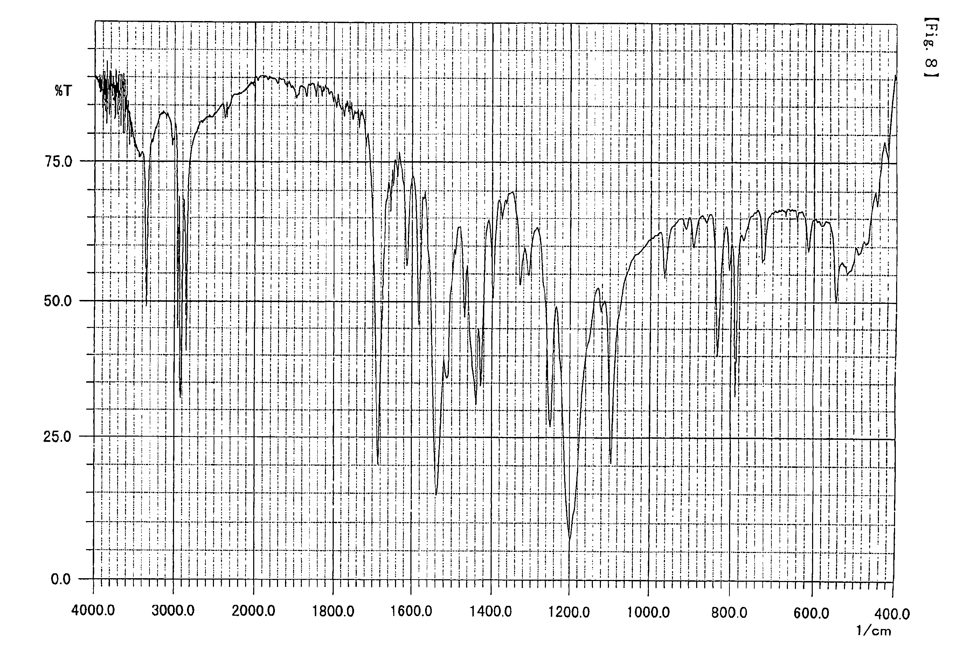

An NMR spectrum chart and an IR spectrum chart of the solid matter are respectively shown in Figure 7 and Figure 8. The solid matter was identified as a compound having the structure represented by formula (5).

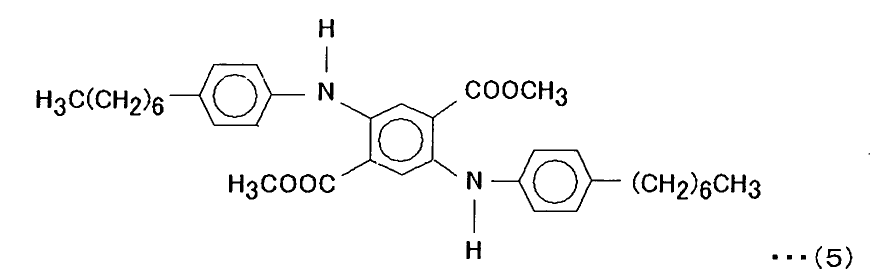

Then, in a 1000 ml three-necked flask were placed 1.5 g of the compound represented by formula (5), 450 ml of 1,4-dioxane, and 10 ml of a 20% aqueous solution of potassium hydroxide. The flask containing the solution was placed in a silicone oil bath and the solution was heated to 110°C. The reaction was allowed to continue for 2 hours. After the termination of the reaction, the solution in the flask was cooled naturally. The pH of the solution was adj usted to a value between 3 and 4. Then, the solution was extracted with chloroform, and the extract was filtered. The filtrate was concentrated and dried up to a purple solid matter.

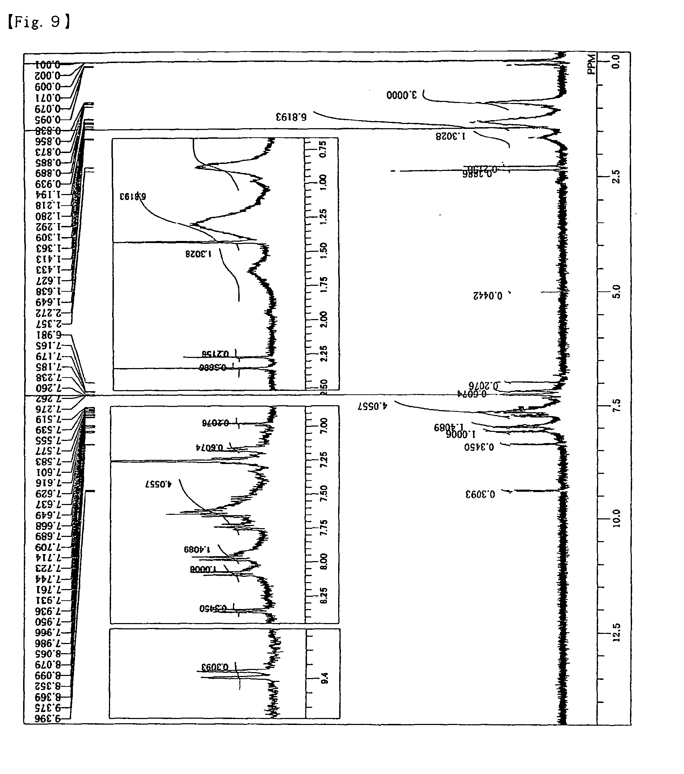

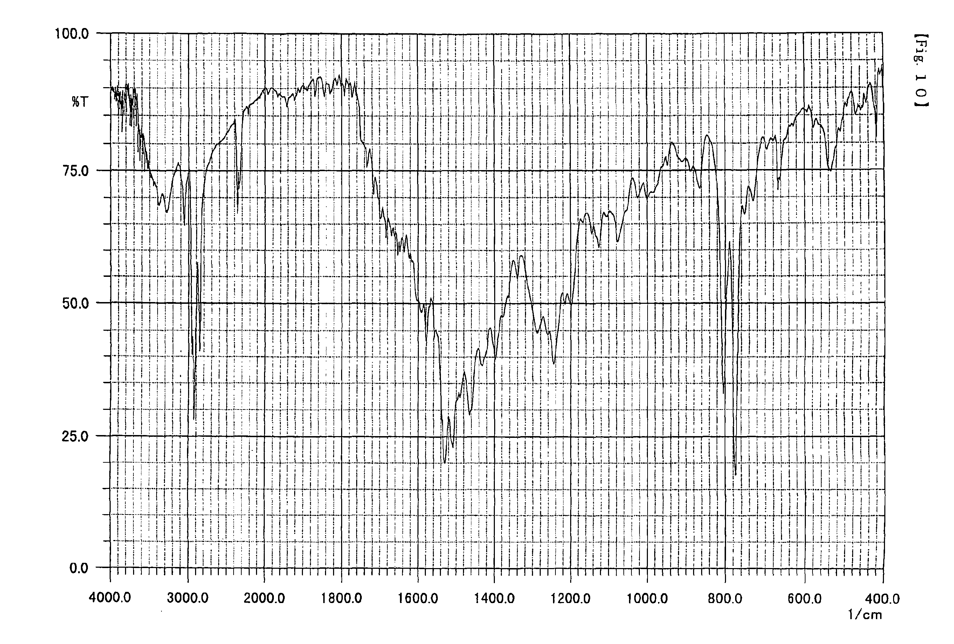

An NMR spectrum chart and an IR spectrum chart of the solid matter are respectively shown in Figure 9 and Figure 10. The solid matter was identified as a dicarboxylic acid having the structure represented by formula (6).

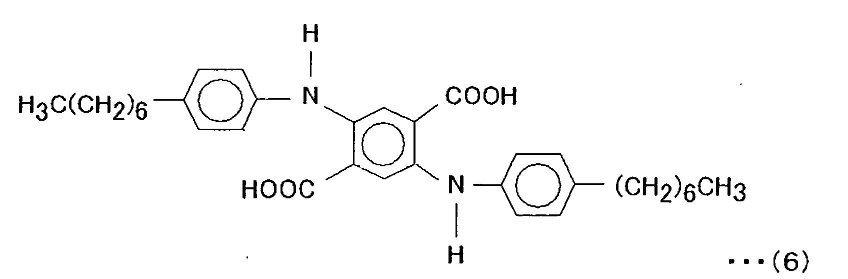

Next, in a 300 ml pear-shaped flask were placed 1.5 g of the dicarboxylic acid represented by formula (6), 100 ml of 1,4-dioxane, 0.22 g of pyridine, and 50 ml of thionyl chloride. The flask containing the solution was placed in a silicone oil bath and the solution was heated to 105°C. The reaction was allowed to continue for 3 hours. After the termination of the reaction, the solution was subjected to vacuum distillation. The liquid obtained as a result of the vacuum distillation was dissolved in 150 ml of tetrahydrofuran. The obtained solution was filtered. Then, the filtrate was concentrated and dried up to a solid matter.

The solid matter was analyzed with NMR spectroscopy and IR spectroscopy. From the obtained data, the solid matter was identified as an acid halide having the structure represented by formula (7).

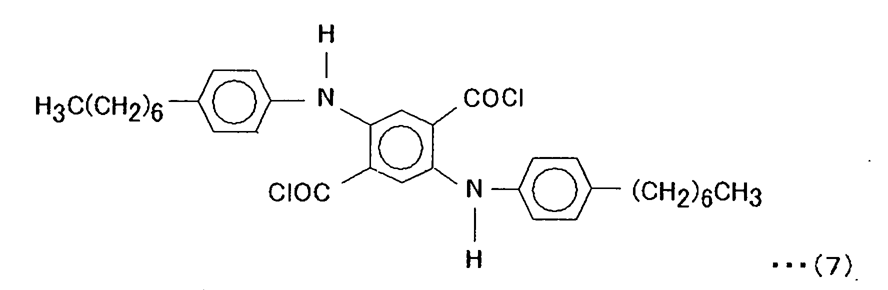

In a 500 ml three-shaped flask were placed 2.3 g of the obtained acid halide, 2.21 g of 1-hydorazinocarbonylnaphthalene, 200 ml of tetrahydrofuran, and 0.64 ml of pyridine. The flask containing the solution was placed in a silicone oil bath and the solution was heated to 60°C. The reaction was allowed to continue for 1.5 hours. After the termination of the reaction, the solution was cooled naturally. The solution was extracted with chloroform. Then, the extract was filtered. The filtrate was concentrated and dried up. A solid matter was obtained.

The solid matter was analyzed with NMR spectroscopy and IR spectroscopy. From the obtained data, the solid matter was identified as a compound having the structure represented by formula (8).

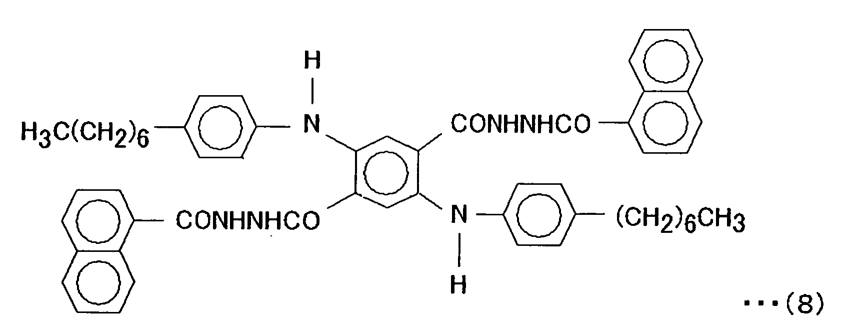

Next, in a 500 ml pear-shaped flask were placed 3.4 g of the compound having the structure represented by formula (8) and 100 ml of phosphoryl chloride. The flask containing the solution was placed in a silicone oil bath and the solution was heated to 105°C. The reaction was allowed to continue for 12 hours. After the termination of the reaction, the solution was cooled naturally. The solution was introduced into ice water. Then, the resultant was filtered and solids were separated. The separated solids were dissolved in toluene and the solution was filtered again. The filtrate was concentrated and dried up. A solid matter was obtained.

An NMR spectrum chart and an IR spectrum chart of the solid matter are respectively shown in Figure 9 and Figure 10. The solid matter was identified as a targeted compound having the structure represented by formula (9).

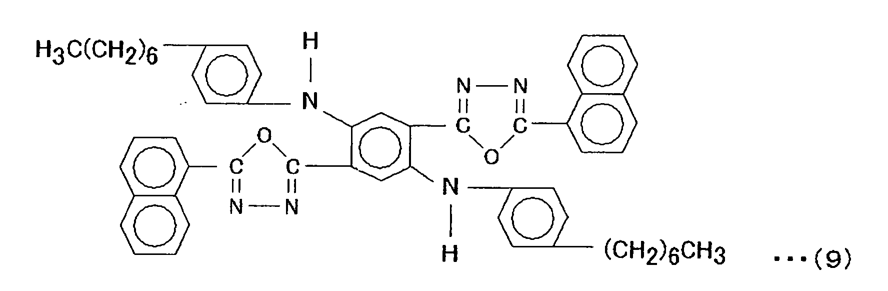

A sample solution was prepared by dissolving the targeted compound represented by formula (9) in toluene. This sample solution was loaded in a model F-4500 spectrofluorophotometer, a product by Shimadzu Corporation, and the fluorescence spectrum of the solution was measured under the following conditions. The measured spectrum is shown in Figure 11.

### Conditions of Measurement

Measuring mode: Wavelength scanning
Exciting wavelength: 365 nm
Wavelength at which the emission of fluorescence started: 380 nm
Wavelength at which the emission of fluorescence ended: 700 nm
Scanning speed: 1200 nm/min.
Slit on the side of excitation: 2.5 nm
Slit on the side of fluorescence emission: 2.5 nm
Photomal voltage: 700 V

As understood from Figure 11, the compound having the structure represented by formula (9) obtained in this example has a fluorescence emission at around 400 nm and from 500 nm to 570 nm. These data confirm that the obtained is a compound that emits white light.

### Industrial Applicability

The luminescent element emitting white light in accordance with the present invention, when it is shaped to a planar one with a large area, maybe used as a planar illuminator, for example a large-area wall illuminator when fixed on a wall, or a large-area ceiling illuminator when fixed on a ceiling. This luminescent element may be utilized for a planar light source in place of a point light source, such as a conventional bulb, and a line light source, such as a conventional fluorescent lamp. In particular, this illuminator can suitably be used to light up walls, ceilings and floors in dwelling rooms, offices and passenger trains, or to make them emit light. Moreover, this luminescent element emitting white light may be suitable for the backlight used in displays of computers, cellular phones and ATMs . Furthermore, this illuminator may be used for various light sources, such as the light source of direct illumination and that of indirect illumination. Also, it may be used for the light sources of advertisement apparatuses, road traffic sign apparatuses and light-emitting billboards, which have to emit light at night and provide good visibility. In addition, because this luminescent element does not include mercury, it is an ecological light source and may be a substitute for conventional fluorescent lamps.

## Claims

1. A white light-emitting compound having the structure represented by formula (1): 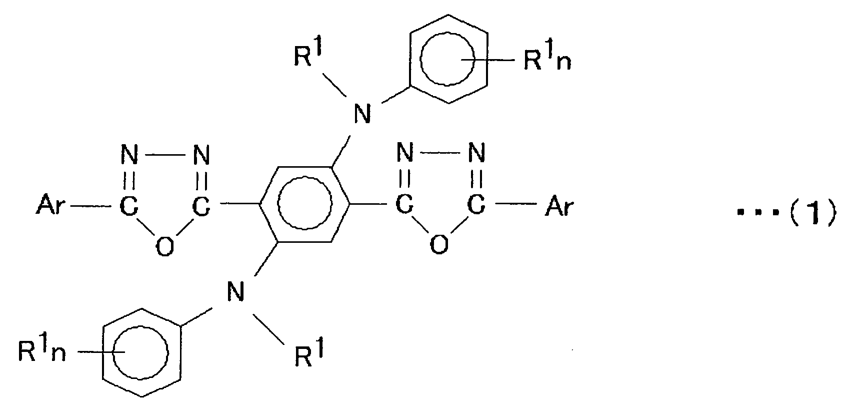 wherein R¹ is hydrogen atom, an alkyl group having 1 to 10 carbon atoms, hydrogen atoms of which may be replaced with fluorine atoms, an alkoxyl group, or an aryl group; Ar denotes a substituent shown by one of formulas (1-1) to (1-6); and n is an integer from 1 to 5, wherein the number of R¹' s which one of the two phenyl groups in formula (1) has is the same as that of R¹' s which the other phenyl group has, and R¹ or R¹' s on one phenyl group are the same as R¹ or R¹' s on the other phenyl group; R¹ bonded with one of the two nitrogen atoms which are bonded with the respective phenyl groups and the central benzene ring is the same as R¹ bonded with the other nitrogen atom; the two Ar's are the same; and the R¹'s bonded with the nitrogen atoms may be different from the R¹'s that the phenyl groups have,
wherein formula (1-1) is: 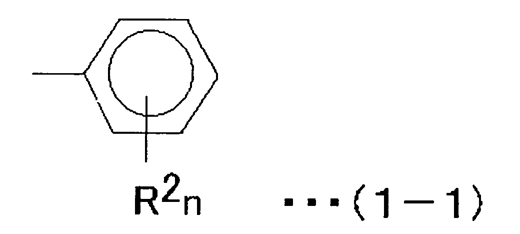 wherein R² means hydrogen atom, or an alkyl group having 1 to 10 carbon atoms, hydrogen atoms of which may be replaced with fluorine atoms; and n denotes the same as above,
formula (1-2) is: 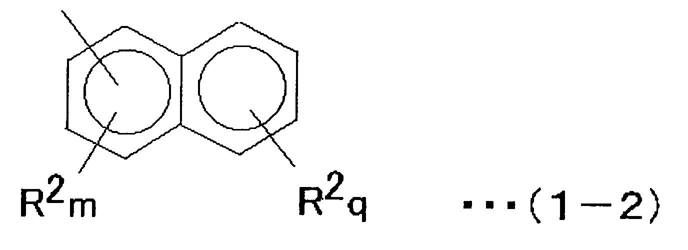 wherein R² means the same as above; m denotes an integer from 1 to 3; q denotes an integer from 1 to 4; and R²' s may be the same or different from each other,
formula (1-3) is: 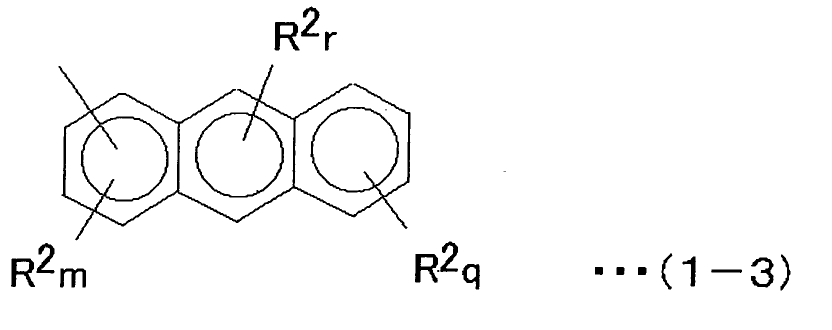 wherein R², m, and q mean the same as above; r denotes an integer of 1 or 2; and R²'s may be the same or different from each other,
formula (1-4) is: 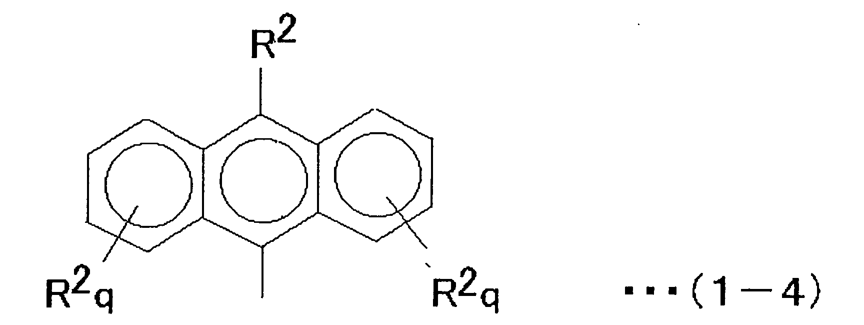 wherein R² and q mean the same as above; and R²' s may be the same or different from each other,
formula (1-5) is: 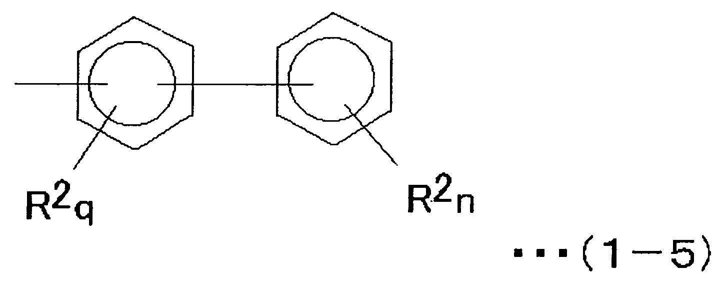 wherein R², n, and q mean the same as above; and R²' s may be the same or different from each other,
formula (1-6) is: 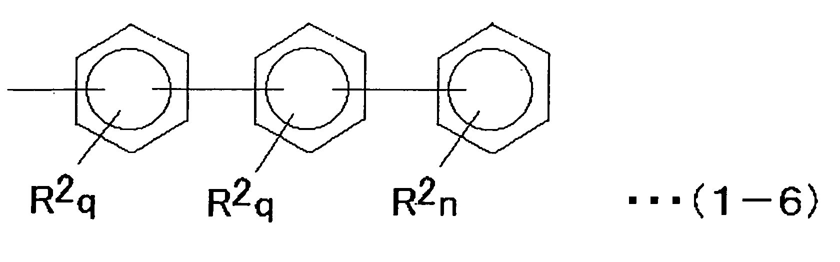 wherein R², n, and q mean the same as above; and R²'s may be the same or different from each other.

2. A process for producing the white light-emitting compound which comprises:
preparing a first compound (c), in accordance with Reaction Formula 1, 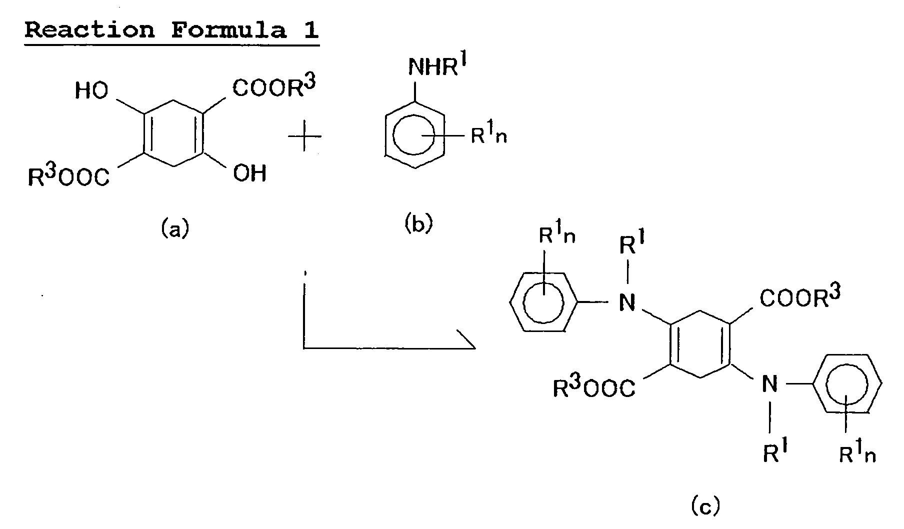 by heating 1,4-dihydroxy-2,5-dialkylcarboxy-2,4-cyclohexadiene (a) , wherein R³ of the compound (a) means an alkyl group, and an aniline derivative (b), wherein R¹ of the aniline derivative (b) is hydrogen atom, an alkyl group having 1 to 10 carbon atoms, hydrogen atoms of which may be replaced with fluorine atoms, an alkoxyl group, or an aryl group, and n denotes an integer of 1 to 5, in a solvent, thereby eliminating a water molecule made from the hydrogen atom bonded to the nitrogen atom of the aniline derivative (b) and the hydroxyl group that 1,9-dihydroxy-2,5-dialkylcarboxy-2,4-cyclohexadiene (a) has;
preparing a second compound (d), in accordance with Reaction Formula 2, 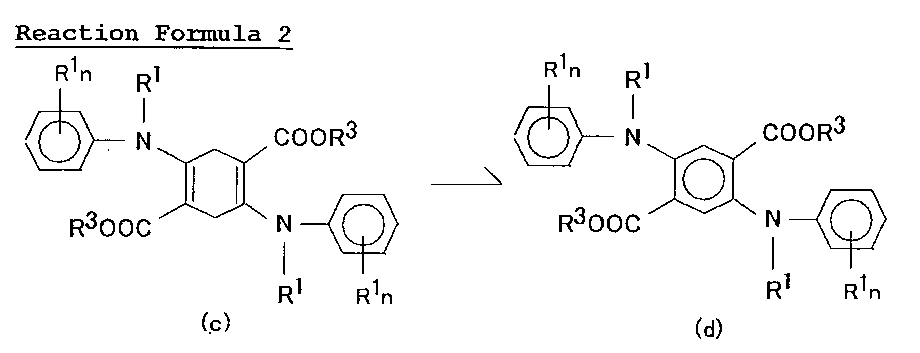 by heating the first compound (c) in a solvent in the presence of a dehydrogenating catalyst to subject the first compound (c) to a dehydrogenation reaction;
preparing a dicarboxylic acid compound (e) , in accordance with Reaction Formula 3, 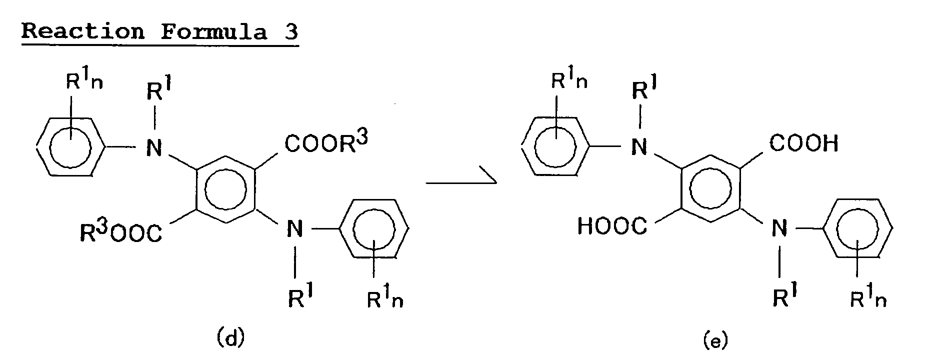 by heating the second compound (d) in a solvent, thereby replacing R³'s of the second compound (d) with hydrogen atoms;
preparing an acid halide (f), in accordance with Reaction Formula 4, 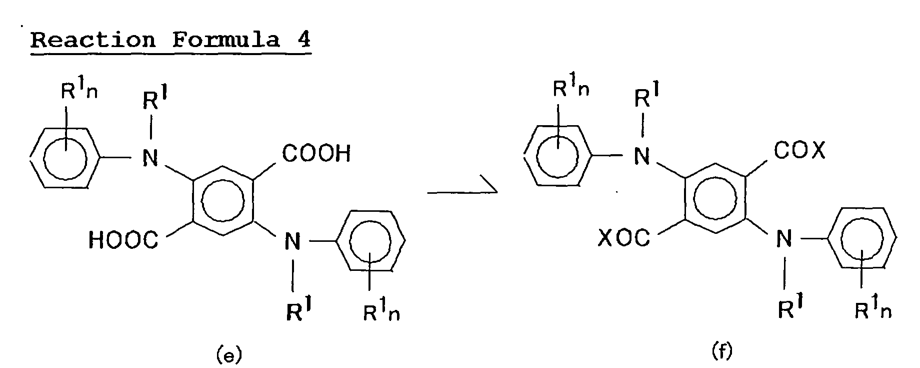 by heating the dicarboxylic acid compound (e) withahalogenating agent in a solvent, thereby replacing the hydroxyl groups of the carboxylic groups with halogen atoms (X);
preparing a third compound (g), in accordance with Reaction Formula 5, 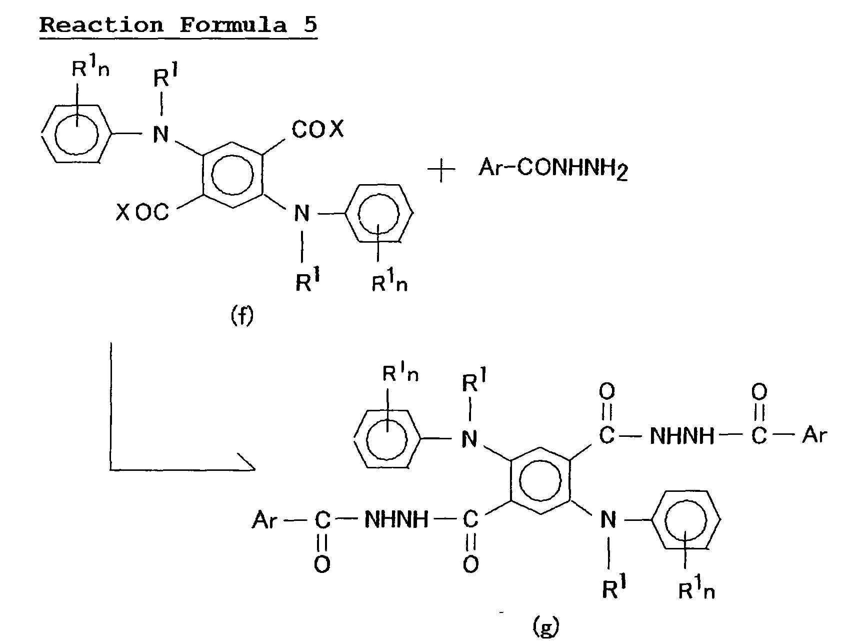 from the acid halide (f) and a hydrazide in which Ar means the same as that described in relation to formula (1); and
preparing a white light-emitting compound (h), in accordance with Reaction Formula 6, 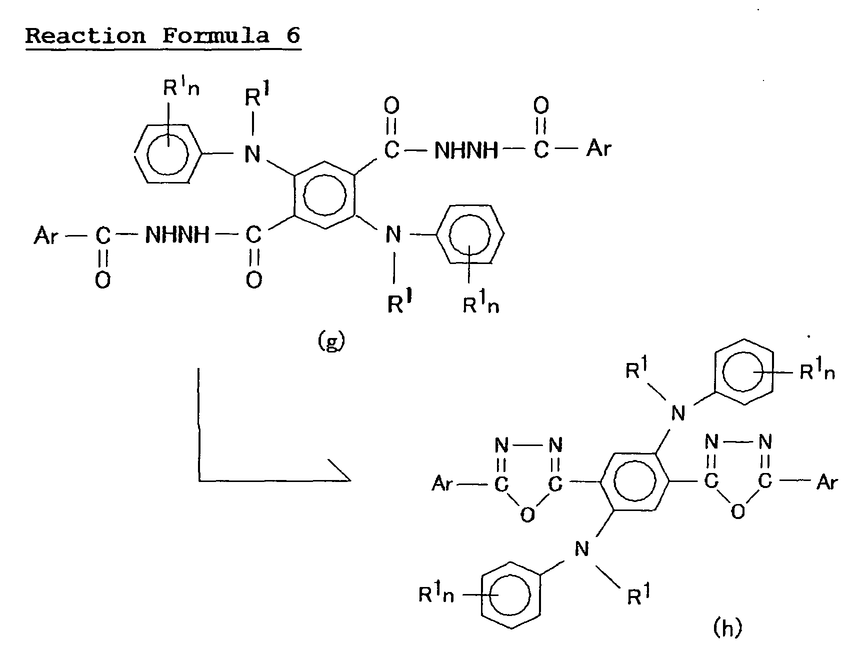 by heating the third compound (g) in a solvent, thereby making the rings in the molecule.

3. A layered article including the white light-emitting compound of claim 1.

4. A layered article according to claim 3 in a form of a luminescent element comprising a pair of electrodes and a light-emitting layer sandwiched between the electrodes, wherein the white light-emitting compound is included in the light-emitting layer.
